(19) Europäisches Patentamt
European Patent Office
Office européen des brevets

(11) Veröffentlichungsnummer: **0 052 225**
**B1**

(12) # EUROPÄISCHE PATENTSCHRIFT

(45) Veröffentlichungstag der Patentschrift: **30.05.84**

(51) Int. Cl.³: **C 07 D 405/06**, C 07 D 403/06

(21) Anmeldenummer: **81108081.1**

(22) Anmeldetag: **08.10.81**

(54) **2-(2'-(Hydanto-5-yl)-äthyl)-5,5-dimethyl-1,3-dioxan, Verfahren zu seiner Herstellung und seine Verwendung.**

(30) Priorität: **15.11.80 DE 3043250**

(43) Veröffentlichungstag der Anmeldung: **26.05.82 Patentblatt 82/21**

(45) Bekanntmachung des Hinweises auf die Patenterteilung: **30.05.84 Patentblatt 84/22**

(84) Benannte Vertragsstaaten: **BE CH DE FR GB LI NL**

(56) Entgegenhaltungen:
**DE - A - 2 930 196**

**Bulletin of the Chemical Society of Japan Vol. 41, Seite 2975-2978 (1968)**
**Angew. Chem. 90, Seite 187-194 (1978)**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Dipl.Chem., Greifenhagenstrasse 25, D-6450 Hanau 9 (DE)**
Erfinder: **Samson, Marc, Dr., Grünaustrasse 1, D-6450 Hanau 9 (DE)**

**EP 0 052 225 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist 2-[2′(Hydanto-5-yl)-äthyl]-5,5-dimethyl-1,3-dioxan der Formel

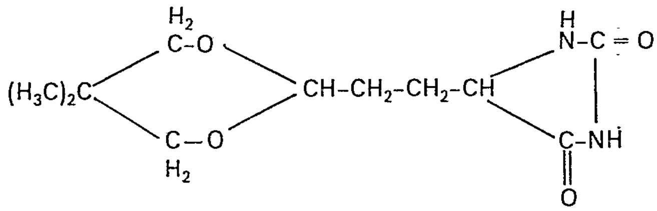

(I)

und ein Verfahren zu seiner Herstellung.

2-[2′-(Hydanto-5-yl)-äthyl]-5,5-dimethyl-1,3-dioxan ist ein wertvolles Zwischenprodukt für die Herstellung von Tryptophan-hydantoin. Ein weiterer Gegenstand der Erfindung ist daher dessen Verwendung zur Herstellung von Tryptophan-hydantoin.

Tryptophan ist ein essentielle Aminosäure, die häufig in Futtermitteln und Mischfuttern die limitierende Aminosäure darstellt. Da Tryptophan durch alkalische Hydrolyse von Tryptophan-hydantoin gewonnen werden kann, kommt dessen Synthese eine grosse Bedeutung zu.

Es sind bereits Verfahren zur Herstellung von Tryptophan-hydantoin bekannt, die letztlich entweder von Acrolein oder von Acrylnitril ausgehen und über mehrere Stufen verlaufen. Die bekannten Verfahren sind jedoch nicht voll befriedigend, weil sie entweder den Einsatz von schwer zugänglichen Reaktionsteilnehmern bzw. Hilfsstoffen erfordern oder in mindestens einer Reaktionsstufe nur verhältnismässig geringe Umsätze ergeben.

So ist aus «Bulletin of the Chemical Society of Japan, Vol. 41, 2975 bis 2978 (1968)» beispielsweise ein Verfahren bekannt, das von Acrolein ausgeht und über Acrolein-diacetat, 1,1-Diacetoxy-4,4-diäthoxy-butan und 5-(γ,γ-Diäthoxypropyl)-hydantoin zum Tryptophan-hydantoin führt. In den beiden letzten Verfahrensstufen beträgt jedoch die Gesamtausbeute nur 62,1% der Theorie.

Aus «Angew. Chem. 90, 187 bis 194 (1978)» ist ferner beispielsweise ein Verfahren bekannt, das von Acrylnitril ausgeht und über β-Cyano-propionaldehyd,5-(β-Cyanoäthyl)-hydantoin,5-(β-Formyläthyl)-hydantoin und dessen Phenyl-hydrazon zum Tryptophan-hydantoin führt. Dieses Verfahren erfordert jedoch insgesamt fünf Verfahrensstufen.

Das erfindungsgemässe 2-[2′-(Hydanto-5-yl)-äthyl]-5,5-dimethyl-1,3-dioxan kann auf einfache Weise und mit guter Ausbeute dadurch hergestellt werden, das man 2-(2′-Formyl-äthyl)-5,5-dimethyl-1,3-dioxan der Formel

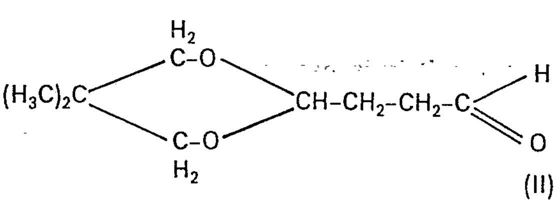

(II)

in wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung umsetzt.

Das als Ausgangsmaterial dienende 2-(2′-Formyläthyl)-5,5-dimethyl-1,3-dioxan kann ebenfalls leicht und mit guter Ausbeute durch Hydroformylierung von 2-Vinyl-5,5-dimethyl-1,3-dioxan gewonnen werden, das seinerseits auch durch Umsetzung von Acrolein mit 2,2-Dimethylpropandiol-1,3 gut zugänglich ist.

Andererseits lässt sich 2-[2-(Hydanto-5-yl)-äthyl]-5,5-dimethyl-1,3-dioxan in Gegenwart von Säuren mit Phenylhydrazin leicht und mit guter Ausbeute direkt in Tryptophan-hydantoin umwandeln. Es stellt daher ein Schlüsselprodukt in einem neuen, von Acrolein ausgehenden, vorteilhaften und kostengünstigen Verfahren zur Herstellung von Tryptophan-hydantoin und von Tryptophan selbst dar.

Zur Herstellung des 2-[2′-(Hydanto-5-yl)-äthyl]-5,5-dimethyl-1,3-dioxan wird 2-(2′-Formyl-äthyl)-5,5-dimethyl-1,3-dioxan in der für die Bildung von Hydantoinen aus Aldehyden an sich bekannten Weise mit Cyanwasserstoff oder einer Cyanidionen liefernden Verbindung, wie Natriumcyanid oder Kaliumcyanid, mit Ammoniak oder einer Ammoniumionen liefernden Verbindung, wie Ammoniumhydroxid oder Ammoniumchlorid, und mit Kohlendioxid oder einer Carbonationen liefernden Verbindung, wie Natrium- oder Kaliumbicarbonat, -carbonat oder -carbamat umgesetzt. Es können auch Verbindungen eingesetzt werden, die gleichzeitig Cyanid- und Amoniumionen liefern, wie Ammoniumcyanid, oder die gleichzeitig Ammonium- und Carbonationen liefern, wie Ammoniumcarbonat und Ammoniumcarbamat.

Die Umsetzung erfolgt in einem Gemisch aus Wasser und Methanol oder Äthanol. Sie kann in einem weiten Temperaturbereich vorgenommen werden. Bevorzugt wird eine Temperatur zwischen 35 und 90°C, weil in diesem Bereich eine zufriedenstellende Reaktionsgeschwindigkeit erreicht wird und der eventuell erforderliche Überdruck technisch kein Hindernis bildet.

Die Mengen der einzelnen Reaktionsteilnehmer können innerhalb weiter Grenzen variiert werden. Vorzugsweise werden je Mol 2-(2′-Formyläthyl)-5,5-dimethyl-1,3-dioxan 1 bis 1,5 Mol Cyanwasserstoff oder einer Cyanidionen liefernden Verbindung, 2 bis 15 Mol Ammoniak oder einer Ammoniumionen liefernden Verbindung und 1 bis 2 Mol Kohlendioxid oder einer Carbonationen liefernden Verbindung eingesetzt. Das 2-(2'-Formyläthyl)-5,5-dimethyl-1,3-dioxan kann gleichzeitig mit allen drei anderen Reaktionsteilnehmern umgesetzt werden. Ebenso ist es aber auch möglich, es zunächst nur mit der Cyanidkomponente zum entsprechenden Cyanhydrin und dieses dann mit den beiden anderen Komponenten gleichzeitig, oder zunächst nur mit der Ammoniumkomponente und dann erst mit der Kohlendioxid- bzw. Carbonatkomponente umzusetzen. Besonders vorteilhaft ist es, wenn das 2-(2'-Formyläthyl)-5,5-dimethyl-1,3-dioxan in Methanol oder Äthanol gelöst und diese Lösung langsam zu einer auf die gewünschte Reaktionstemperatur erwärmten wässrigen Lösung oder Suspension der anderen Reaktionsteilnehmer zudosiert wird. Zur Erzielung eines hohen Umsatzes ist eine angemessene Nachreaktionszeit von beispielsweise etwa 5 Stunden nach dem Ende der Dosierung zu empfehlen.

Da das gebildete 2-[2'-(Hydanto-5-yl)-äthyl]-5,5-dimethyl-1,3-dioxan bei niedrigen Temperaturen in Wasser schwerlöslich ist, kann es nach Abdestillieren des Alkohols durch Abkühlen des Reaktionsgemisches auf eine Temperatur unter 25°C praktisch vollständig und in hoher Reinheit abgeschieden werden.

Die nach der Abtrennung des 2-[2'-(Hydanto-5-yl)-äthyl]-5,5-dimethyl-1,3-dioxans verbleibende Mutterlauge enthält unter Umständen noch eine gewisse Menge des als Nebenprodukt gebildeten α-N-Carbamoyl-carbonsäureamids der Formel

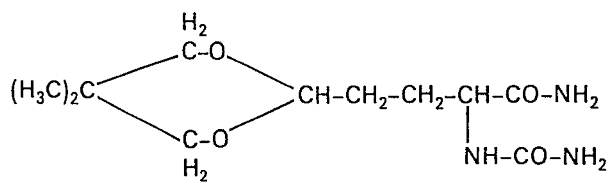

(III)

gelöst. Dieses kann bei einem pH unter 7 leicht in zusätzliches Hydantoin umgewandelt werden. Alternativ kann aber auch die Mutterlauge, gegebenenfalls nach Einengen, im Kreislauf zurückgeführt und als Lösungsmittel für einen neuen Ansatz verwendet werden.

Um aus dem 2-[2'-(Hydanto-5-yl)-äthyl]-5,5-dimethyl-1,3-dioxan Tryptophan-hydantoin herzustellen, wird es bei einem pH zwischen 0,1 und 4, vorzugsweise zwischen 1 und 3, mit Phenyl-hydrazin umgesetzt. Der erforderliche pH kann durch eine anorganische Säure, wie Schwefelsäure oder Phosphorsäure, durch eine organische Säure, wie Oxalsäure, Ameisensäure, Essigsäure, Benzolsulfonsäure oder p-Toluolsulfonsäure, oder durch einen stark sauren Ionenaustauscher eingestellt werden. Bevorzugt wird die Verwendung von Salzsäure. Da bei der Bildung des Tryptophan-hydantoins aus dem 2-[2'-(Hydanto-5-yl)-äthyl]-5,5-dimethyl-1,3-dioxan pro Mol ein Mol Ammonaik freigesetzt wird, ist es unter Umständen erforderlich, während der Umsetzung durch Zugabe von Säure den pH-Wert nachzustellen. Die Reaktionstemperatur kann innerhalb weiter Grenzen variiert werden. Zweckmässig sind Temperaturen zwischen 60 und 150°C, vorzugsweise zwischen 70 und 120°C.

Das Phenylhydrazin kann im Überschuss eingesetzt werden. Aus wirtschaftlichen Gründen ist es jedoch vorteilhafter, nur die dem eingesetzten 2-[2'-(Hydanto-5-yl)-äthyl]-5,5-dimethyl-1,3-dioxan äquivalente Menge anzuwenden.

Das Phenylhydrazin kann mit dem 2-[2'-(Hydanto-5-yl)-äthyl]-5,5-dimethyl-1,3-dioxan und der erforderlichen Menge an Säure vermengt und auf die gewünschte Reaktionstemperatur erhitzt werden. Ebenso ist es aber auch möglich, eine saure Lösung des Phenylhydrazins vorzulegen, zu erhitzen und eine Lösung des 2-[2'-(Hydanto-5-yl)-äthyl]-5,5-dimethyl-1,3-dioxans zuzudosieren. In beiden Fällen ist eine Reaktionszeit von insgesamt etwa 3 bis 4 Stunden im allgemeinen ausreichend. Nach beendeter Reaktion kann die erhaltene Lösung des Tryptophan-hydantoins eingeengt und der Rückstand umkristallisiert werden. Man kann aber auch durch Abkühlen der Lösung das Tryptophan-hydantoin zur Kristallisation bringen und abtrennen. Die Reinheit des so erhaltenen Produkts liegt normalerweise über 95%. Durch Umkristallisieren erhält man reines Tryptophan-hydantoin mit einem Schmelzpunkt von 213 bis 216°C.

Durch die nachfolgenden Beispiele soll die Erfindung näher verdeutlicht werden. Sofern nicht anders angegeben, bedeuten die Prozentangaben Gewichtsprozente.

Beispiel 1

In einem mit Rührer und Rückflusskühler versehenen Kolben werden 1090 g wässriger Ammoniak (25%ig), 288 g Ammonium-carbonat, 180 g Wasser und 95 ml flüssiger Cyanwasserstoff eingebracht. Das Gemisch wird auf 40°C erwärmt. Bei dieser Temperatur werden innerhalb von einer Stunde 344 g 2-[2'-(Formyläthyl)-5,5-dimethyl-1,3-dioxan, gelöst in 500 ml Methanol, zugegeben, wonach die Temperatur noch 5 Stunden lang auf 40°C gehalten wird. Anschliessend werden das Methanol abdestilliert und durch weiteres Erhitzen auf 100°C die Ammoniumsalze verkocht. Beim langsamen Abkühlen bis auf 20°C kristallisieren 388 g 2-[2'-(Hydanto-5-yl)-äthyl]-5,5-dimethyl-1,3-dioxan (80% der Theorie) aus. Schmelzpunkt: 168–171°C.

Elementaranalyse: $C_{11}H_{18}N_2O_4$

Berechnet: C 54,53% H 7,49% N 11,56%
gefunden: C 54,18% H 7,30% N 11,81%

IR-Spektrum: 3500–3000 $cm^{-1}$; 1785 $cm^{-1}$; 1740 $cm^{-1}$

$^1H$-NMR-Spektrum (DMSO d6-$CDCl_3$):

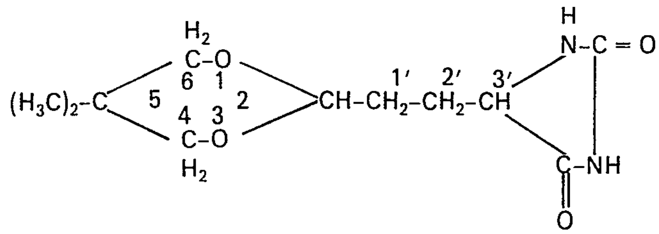

δ = 0,70 (s, 3H): 5-$CH_3$
δ = 1,10 (s, 3H): 5-$CH_3$
δ = 1,3–1,9 (m, 4H): H-1′, H-2′
δ = 3,4 (q, 4H): H-4, H-6
δ = 3,95 (m, 1H): H-3
δ = 4,5 (t, 1H): H-2
δ = 7,9 (s, 1H): NH
δ = 10,5 (s, 1H): NH

Beispiel 2

In einen mit einem Rührer versehenen Autoklaven werden 680 g wässriger Ammoniak (25%ig), 288 g Ammoniumcarbonat und 390 ml Wasser eingebracht. Anschliessend werden unter Rühren 116 ml Cyanwasserstoff zugegeben. Danach wird bei einer Temperatur von etwa 35°C innerhalb einer Stunde eine Lösung von 344 g 2-(2′-Formyläthyl)-5,5-dimethyl-1,3-dioxan in 500 ml Methanol unter Rühren zugesetzt. Das Gemisch wird 3 Stunden lang auf 70°C gehalten. Dann werden das Methanol abdestilliert und durch weiteres Erhitzen auf 100°C die Ammoniumsalze verkocht. Beim langsamen Abkühlen auf Raumtemperatur kristallisieren 411 g (85% der Theorie) 2-[2′-(Hydanto-5-yl)-äthyl]-5,5-dimethyl-1,3-dioxan aus. Schmelzpunkt: 169–171°C.

Beispiel 3

Zu einer Lösung von 26 g Kaliumcyanid in 150 ml Wasser wird im Verlaufe einer Stunde eine Lösung von 68,8 g 2-(2′-Formyläthyl)-5,5-dimethyl-1,3-dioxan in 150 ml Methanol zugetropft und es wird noch eine Stunde lang bei 25°C nachgerührt. Anschliessend wird das Reaktionsgemisch im Verlaufe einer Stunde bei 35 bis 40°C in eine gerührte Suspension aus 76,8 g Amoniumcarbonat und 200 ml wässrigem Ammoniak (25%ig) zugetropft und es wird noch 5 Stunden lang bei 40°C nachgerührt. Dann wird das Methanol abdestilliert, die Amoniumsalze werden verkocht und das Reaktionsgemisch wird bei 100°C auf 180 ml Gesamtvolumen eingeengt. Beim Abkühlen auf Raumtemperatur kristallisieren 81,3 g (84% der Theorie) 2-[2′-(Hydanto-5-yl)-äthyl]-5,5-dimethyl-1,3-dioxan aus.

Beispiel 4

Eine 80°C warme Lösung von 24,2 g 2-[2′-(Hydanto-5-yl)-äthyl]-5,5-dimethyl-1,3-dioxan, hergestellt nach einem der Beispiele 1 bis 3, in 250 ml 0,1 N HCl wird innerhalb einer Stunde in eine 90°C warme gerührte Lösung von 9,9 g Phenylhydrazin in 100 ml 0,1 N HCl zugetropft. Das Reaktionsgemisch wird anschliessend noch 2,5 Stunden lang auf 90°C gehalten. Nach langsamem Abkühlen auf 10°C wird der entstandene Niederschlag abfiltriert, mit Wasser nachgewaschen und bei vermindertem Druck getrocknet. Die Ausbeute an Tryptophan-hydantoin beträgt 18,9 g (82,5% der Theorie).

Elementaranalyse: $C_{12}H_{11}N_3O_2$

Berechnet: C 62,9% H 4,8% N 18,3%
gefunden: C 61,6% H 4,9% N 18,1%

Beispiel 5

Eine Suspension aus 96,8 g 2-[2′-(Hydanto-5-yl)-äthyl]-5,5-dimethyl-1,3-dioxan, 57,8 g Phenylhydrazin-hydrochlorid, 50 ml 2 N HCl und 1200 ml Wasser wird 3 Stunden lang unter kräftigem Rühren auf 90°C erhitzt. Nach langsamem Abkühlen auf 10°C wird der entstandene Niederschlag abfiltriert und bei vermindertem Druck getrocknet. Die Ausbeute an Tryptophan-hydantoin beträgt 75,5 g (78% der Theorie).

**Patentansprüche**

1. 2-[2′-(Hydanto-5-yl)-äthyl]-5,5-dimethyl-1,3-dioxan der Formel

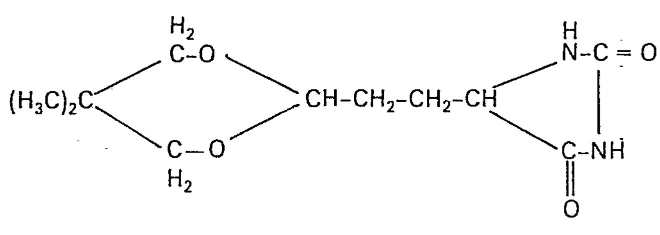

(I)

2. Verfahren zur Herstellung von 2-[2'-(Hydanto-5-yl)-äthyl]-5,5-dimethyl-1,3-dioxan der Formel (I), dadurch gekennzeichnet, dass man 2-(2'-Formyläthyl)-5,5-dimethyl-1,3-dioxan der Formel

(II)

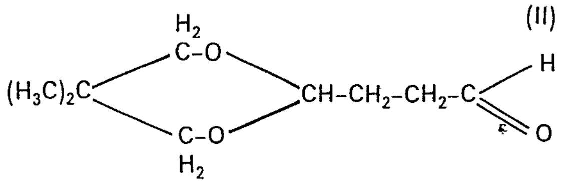

in wässrig-alkoholischer Lösung mit Cyanwasserstoff oder einer Cyanidionen liefernden Verbindung, Ammoniak oder einer Ammoniumionen liefernden Verbindung und Kohlendioxid oder einer Carbonationen liefernden Verbindung umsetzt.

3. Verfahren nach Anspruch 2, dadurch gekennzeichnet, dass man den Cyanwasserstoff oder die Cyanidionen liefernde Verbindung in einer Menge zwischen 1 und 1,5 Mol, den Ammoniak oder die Ammoniumionen liefernde Verbindung in einer Menge zwischen 2 und 15 Mol und das Kohlendioxid oder die Carbonationen liefernde Verbindung in einer Menge zwischen 1 und 2 Mol, jeweils pro Mol eingesetztem 2-(2'-Formyläthyl)-5,5-dimethyl-1,3-dioxan, einsetzt.

4. Verfahren nach Anspruch 2 oder 3, dadurch gekennzeichnet, dass man die Umsetzung bei einer Temperatur zwischen 35 und 90°C durchführt.

5. Verwendung von 2-[2'-(Hydanto-5-yl)-äthyl]-5,5-dimethyl-1,3-dioxan der Formel (I) zur Herstellung von Tryptophan-hydantoin.

**Revendications**

1. 2-[2'-(hydanto-5-yl)-éthyl]-5,5-diméthyl-1,3-dioxanne de formule

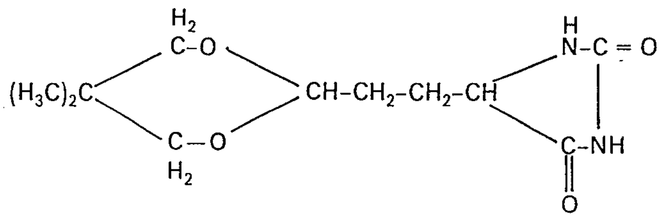

(I)

2. Procédé pour la préparation de 2-[2'-(Hydanto-5-yl)-éthyl]-5,5-diméthyl-1,3-dioxanne de formule (I), caractérisé en ce qu'on fait réagir le 2-(2'-formyléthyl)-5,5-diméthyl-1,3-dioxanne de forumle

(II)

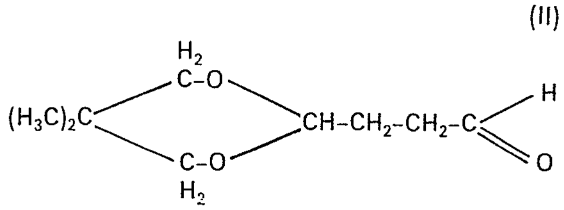

dans une solution aqueuse-alcoolique avec l'acide cyanhydrique ou un composé fournissant des ions cyanure, l'ammoniac ou un composé fournissant des ions ammonium et le dioxyde de carbone ou un composé fournissant des ions carbonate.

3. Procédé selon la revendication 2, caractérisé en ce qu'on introduit l'acide cyanhydrique ou le composé fournissant des ions cyanure en une quantité comprise entre 1 et 1,5 mole, l'ammoniac ou le composé fournissant les ions ammonium en une quantité comprise entre 2 et 15 moles et le dioxyde de carbone ou le composé fournissant les ions carbonate en une quantité comprise entre 1 et 2 moles, chaque fois par mole de 2-(2'-formyléthyl)-5,5-diméthyl-1,3-dioxanne introduit.

4. Procédé selon la revendication 2 ou 3, caractérisé en ce qu'on effctue la réaction à une température comprise entre 35 et 90°C.

5. Utilisation de 2-[2'-(hydanto-5-yl)-éthyl]-5,5-diméthyl-1,3-dioxanne de formule (I) pour la préparation de tryptophane-hydantoïne.

**Claims**

1. 2-[2'-(hydanto-5-yl)-ethyl]-5,5-dimethyl-1,3-dioxane corresponding to the formula

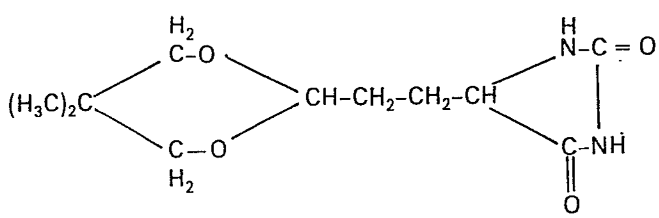

(I)

2. A process for the production of 2-[2'-(hydanto-5-yl)-ethyl]-5,5-dimethyl-1,3-dioxane corresponding to formula (I), characterised in that 2-(2'-formylethyl)-5,5-dimethyl-1,3-dioxane corresponding to the formula

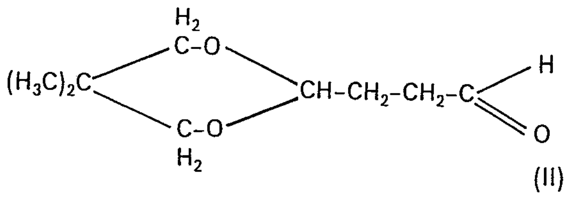

(II)

is reacted in aqueous-alcoholic solution with hydrogen cyanide or a cyanide ion-producing compound, ammonia or an ammonium ion-producing compound and carbon dioxide or a carbonate ion-producing compound.

3. A process according to claim 2, characterised in that the hydrogen cyanide or the cyanide ion-producing compound is used in a quantity of from 1 to 1.5 mols, the ammonia or the ammonium ion-producing compound is used in a quantity of from 2 to 15 mols and the carbon dioxide or the carbonate ion-producing compound is used in a quantity of from 1 to 2 mols, in each case per mol of 2-(2′-formylethyl)-5,5-dimethyl-1,3-dioxane which is used.

4. A process according to claims 2 or 3, characterised in that the reaction is carried out at a temperature of from 35 to 90°C.

5. The use of 2-[2′-(hydanto-5-yl)-ethyl]-5,5-dimethyl-1,3-dioxane corresponding to formula (I) for the production of tryptophane-hydantoin.